# EUROPÄISCHE PATENTSCHRIFT

(11) **EP 0 326 981 B1**
(45) Veröffentlichungstag und Bekanntmachung des Hinweises auf die Patenterteilung: **09.11.1994**
(21) Anmeldenummer: 89101523.2
(22) Anmeldetag: 30.01.1989
(51) Int. Cl.: C07D 471/04, A61K 31/505

(54) **4-Oxo-4H-pyrido[1,2-a]pyrimidin-3-carbonsäureamid-Derivate, deren Herstellung sowie diese Verbindungen enthaltende Arzneimittelpräparate und deren Herstellung**
4-Oxo-4H-pyrido[1,2-a]pyrimidine-3-carboxamide derivatives, their preparation, medicaments containing them and their preparation
Dérivés de 4-oxo-4H-pyrido[1,2-a]pyrimidine 3-carboxamide, leur préparation, médicaments les contenant et leur préparation

(30) Priorität: 03.02.1988 HU 50188
(43) Veröffentlichungstag der Anmeldung: 09.08.1989
(73) Patentinhaber: CHINOIN Gyogyszer és Vegyészeti Termékek Gyára RT., H-1045 Budapest IV (HU)
(72) Erfinder: Hermecz, István, Dr. Dipl.-Ing. Chem., H-1056 Budapest (HU); Knoll, Jozsef, Dr., H-1137 Budapest (HU); Vasvári geb. Debreczy, Lelle, Dipl.-Ing. Chem., H-1122 Budapest (HU); Gyires, Klára, Dr., H-1016 Budapest (HU); Sipos, Judit, Dr., H-1116 Budapest (HU); Horváth, Agnes, Dr. Dipl. Ing. Chem., H-1021 Budapest (HU); Tardos, Lászlo, Dr., H-1111 Budapest (HU); Balogh, Mária, Dr. Dipl.-Ing. Chem., H-2120 Dunakeszi (HU)
(74) Vertreter: Bartsch, Elisabeth, Dr.

(56) Entgegenhaltungen:
- DE-A- 2 913 295
- FR-A- 2 223 036
- GB-A- 1 209 946
- DRUGS UNDER EXPERIMENTAL AND CLINICAL RESEARCH, Band 13, Nr. 5, Mai 1987,Seiten 253-258, Genf, CH; J. KNOLL et al.: "1,6-Dimehtyl-4-oxo-1,6,7,8,9,9alpha-hexahydro-4H-pyrido(1,2-alpha)-pyrimidine-3-carboxamide (CH-127)protects against the intestinal damage in rates caused by two weeks' daily administration of indomethacin"

## Beschreibung

Die Erfindung betrifft neue 4-Oxo-4H-pyrido[1,2-a]pyrimidin-3-carbonsäureamid-Derivate, ihre Säureadditionssalze, Verfahren zu ihrer Herstellung und die diese Verbindungen enthaltenden Arzneimittelpräparate. Die Verbindungen weisen pharmakologische, in erster Linie gastroprotektive Wirkungen auf. Sie können daher in der Medizin in erster Linie als gastroprotektive Mittel gegen Ulcus verwendet werden.

Es ist bekannt, daß manche 4-Oxo-4-H-pyrido[1,2-a]pyrimidin-Derivate schmerzstillende und sonstige cerebrale Wirkungen aufweisen (GB-PS 1 209 946, US-PS 4 291 036, DE-OS 2 414 751). Eine der vorteilhaftesten dieser Verbindungen ist das in der klinischen Praxis als Analgetikum eingesetzte 1,6-Dimethyl-3-äthoxycarbonyl-6-methyl-4-oxo-6,7,8,9-tetrahydro-4H-pyrido[1,2-a]pyrimidiniummethosulfat (Arzneimittelforschung 1972, 22, 815). An anderen Derivaten wurden antiarteriosklerotische (DE-OS 2 705 775), antiallergische und antiasthmatische (belgische Patentschriften 873 192 und 873 194), entzündungshemmende (DS-OS 2 728 198 und 2 526 983; Arzneimittelforschung 1979, 29, 766), cardiovaskuläre (Sankyo Kenkyusho Nempo 1977, 29, 75) und selektiv die Serotonin-2-Rezeptoren blockierende Wirkung (US-PS 4 342 870) beobachtet. Auch über die Wirkung von Tetrahydro- und Hexahydro-4-ozo-4H-pyrido[1,2-a]pyrimidin-3-carbonsäure-Derivaten gegen Ulcus wurde berichtet (Drugs Exptl. Clin. Res. 1985, 11, 493 und 1987, 13, 253). Erwähnt wird die analgetische Wirkung ungesättigter 4-Oxo-4H-pyrido[1,2-a]pyrimidin-3-carbonsäure-Derivate, die jedoch wegen der hydrolytischen Instabilität des Bicyclus nicht genutzt werden konnte (Magyar Kémikusok Lapja 1976, 31, 281).

Die vorliegende Erfindung betrifft nun neue 4-Oxo-4H-pyrido[1,2-a]pyrimidin-3-carbonsäureamid-Derivate der allgemeinen Formel (I), 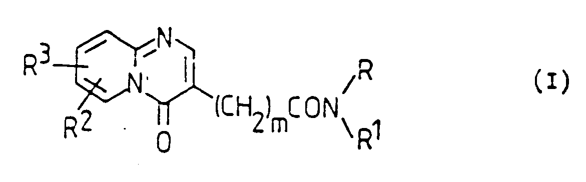
worin
- R: eine Alkylgruppe mit 1-12 Kohlenstoffatomen, die gegebenfalls durch eine Alkoxycarbonylgruppe mit 1-4 Kohlenstoffatomen substituiert sein kann, ferner eine Cycloalkylgruppe mit 3-9 Kohlenstoffatomen, eine Adamantylgruppe oder eine gegebenenfalls substituierte Phenylgruppe, und
- R¹: Wasserstoff oder eine Alkylgruppe mit 1-4 Kohlenstoffatomen bedeuten oder
- R und R¹: unter Bildung der Gruppierung der Formel -(CH₂)ₙ-zusammentreten, worin n 4, 5 oder 6 ist,
- R²: Wasserstoff oder eine Alkylgruppe mit 1-4 Kohlenstoffatomen,
- R³: Wasserstoff oder eine Alkylgruppe mit 1-4 Kohlenstoffatomen, und
- m: 0 oder 1 bedeuten. Die Erfindung betrifft auch die physiologisch verträglichen Salze dieser Verbindungen.

Die Alkylgruppen mit 1-12 beziehungsweise 1-4 Kohlenstoffatomen können gerade oder verzweigt sein (zum Beispiel die Methyl-, Äthyl-, n-Propyl-, Isopropyl-, n-Butyl-, Isobutyl-, sec.-butyl-, tert.-Butyl-, Neopentylgruppe). Unter dem Ausdruck "Cycloalkyl mit 3-9 Kohlenstoffatomen" sind gegebenenfalls durch eine Alkylgruppe substituierte cycloaliphatische gesättigte Kohlenwasserstoffgruppen zu verstehen (zum Beispiel die Cyclopropyl-, Methylcyclopropyl-, Cyclobutyl-, 2,3-Dimethylcyclobutyl-, Cyclohexylgruppe). Die gegebenenfalls substituierte Phenylgruppe kann durch Alkylgruppen mit 1-4 Kohlenstoffatomen, durch Halogen, Alkoxygruppen mit 2-5 Kohlenstoffatomen, Hydroxylgruppen, Trifluormethylgruppen, Cyano, Carboxyl, Alkoxycarbonylgruppen mit 1-4 Kohlonstoffatomen einfach oder mehrfach substituiert sein, beziehungsweise eine Methylendioxybrücke aufweisen. Als Halogenatome kommen Fluor-, Chlor-, Brom- und Jodatome in Frage.

Aus den Verbindungen der allgemeinen Formel (I) können mit physiologisch verträglichen anorganischen und organischen Säuren Salze gebildet werden (zum Beispiel Hydrochloride, Hydrobromide, Hydrojodide, Sulfate, Nitrate, Phosphate, Mesylate, Maleate, Succinate, Acetate, Tartrate, Lactate Fumarate, Citrate, Perchlorate).

Erfindungsgemäß werden die Verbindungen der allgemeinen Formel (I) (worin die Bedeutung von R, R¹, R², R³ und m die gleiche wie oben ist) und ihre physiologisch verträglichen Salze hergestellt, indem man
a) 4-Oxo-4H-pyrido[1,2-a]pyrimidin-3-cärbonsäureester der allgemeinen Formel (II) 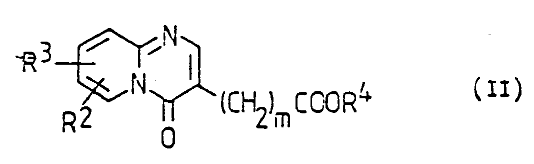 (worin die Bedeutung von R², R³ und m die gleiche wie oben ist und R⁴ eine Alkylgruppe mit 1-4 Kohlenstoffatomen ist) mit Aminen der allgemeinen Formel (III) 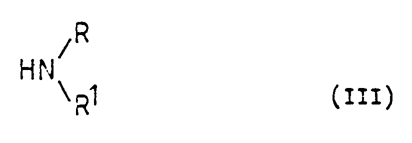 (worin die Bedeutung von R und R¹ die gleiche, wie oben ist) oder
b) gemischte 4-Oxo-4H-pyrido[1,2-a]pyrimidin-3-carbonsäure-anhydride der allgemeinen Formel (IV) 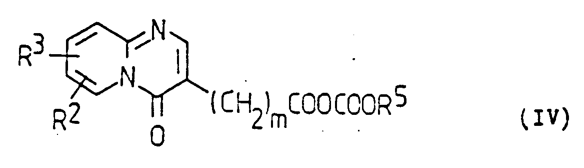 (worin die Bedeutung von R², R³ und m die gleiche, wie oben ist, und R⁵ eine Alkygruppe mit 1-4 Kohlenstoffatomen oder eine Benzylgruppe ist) mit Aminen der allgemeinen Formel (III) umsetzt,
und gewünschtenfalls die erhaltene Verbindung der allgemeinen Formel (I) in an sich bekannter Weise mit einer physiologisch verträglichen Säure zu dem entsprechenden Säureadditionssalz umsetzt oder aus ihrem Salz freisetzt und gewünschtenfalls zu einem anderen Salz umsetzt.

Das Verfahren a) wird vorzugsweise unter Verwendung eines Lösungsmittels ausgeführt. Als Lösungsmittel können die für Amidierungsreaktionen üblichen Lösungsmittel verwendet werden. Die Reaktionstemperatur wird im allgemeinen in Abhängigkeit von den Eigenschaften der Reaktionspartner gewählt. Die Reaktion kann zum Beispiel bei Raumtemperatur oder beim Siedepunkt des Lösungsmittels, aber auch bei davon abweichenden Temperaturen ausgeführt werden. Im allgemeinen arbeitet man bei Normaldruck, jedoch kann die Umsetzung auch bei davon abweichenden Drücken, vorzugsweise bei Überdruck, vorgenommen werden. Findet als Lösungsmittel ein mit wasser mischbares Lösungsmittel (zum Beispiel ein Alkohol) Verwendung, so kann das Reaktionsgemisch eine beliebige Menge Wasser enthalten. Die entstandene Verbindung der allgemeinen Formel (I) fällt, gegebenenfalls nach Verdünnen mit Wasser, aus dem Reaktionsgemisch aus und kann zum Beispiel durch Filtrieren oder durch Eindampfen des Lösungsmittels und Umkristallisieren des Rückstandes isoliert werden.

Beim Verfahren b) wird die Verbindung der allgemeinen Formel (IV) vorzugsweise in situ hergestellt, wobei man von Carbonsäuren der allgemeinen Formel (V) 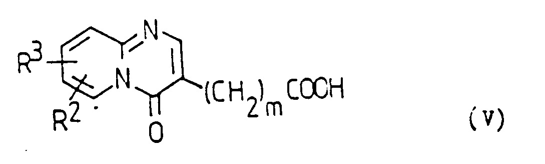
(worin die Bedeutung von R², R³ und m die gleiche wie oben ist) und Chlorameisensäureestern der allgemeinen Formel (VI)

Cl-COOR⁵ (VI)

(worin die Bedeutung von R⁵ die gleiche wie oben ist) ausgeht.

Gemäß einer Ausführungsform des Verfahrens b) wird die Verbindung der allgemeinen Formel (V) in einem organischen Lösungsmittel, vorzugsweise einem chlorierten Kohlenwasserstoff (insbesondere Chloroform) oder einem Äther (insbesondere Dioxan oder Tetrahydrofuran) gelöst und zu der erhaltenen Lösung nach Zusatz eines Trialkylamins, vorzugsweise Triäthylamin oder Tributylamin, bei Temperaturen zwischen -30 und 50 °C, vorzugsweise -20 und 0 °C, tropfenweise die Verbindung der allgemeinen Formel (VI), vorzugsweise Chlorameisensäuremethyl-, Chlorameisensäureäthyl-, Chlorameisensäurebenzyl- oder Chlorameisensäureisopropylester, gegeben. Zu der entstandenen Verbindung der allgemeinen Formel (IV) wird - ebenfalls tropfenweise - das Amin der allgemeinen Formel (III) gegeben, gewünschtenfalls gelöst in einem der oben angegebenen Lösungsmittel, oder in Form seines Säureadditionssalzes, in diesem Falle zusammen mit einem Trialkylamin, vorzugsweise Triäthylamin oder Tributylamin. Das Reaktionsgemisch wird bei der oben genannten Temperatur gerührt, dann läßt man die Temperatur auf Raumtemperatur ansteigen und schüttelt das Reaktionsgemisch zuerst mit wässriger Natriumhydrogencarbonatlösung, dann mit Wasser aus. Nach dem Trocknen des Reaktionsgemisches wird das Lösungsmittel entfernt und der Rückstand aus einem geeigneten Lösungsmittel umkristallisiert.

Sowohl im Verfahren a) wie auch im Verfahren b) kann das Verhältnis der Reaktionspartner beliebig gewählt werden. Vorzugsweise setzt man auf 1 Mol Verbindung der allgemeinen Formel (II) beziehungsweise (IV) 1-10 Mol Amin der allgemeinen Formel (III) ein.

Die ein Stickstoffbrückenkopfatom aufweisenden Ausgangsverbindungen sind zum Teil bekannt. Die Ausgangsverbindungen der allgemeinen Formeln (II) und (V) sind literaturbekannt (Arzneimittelforschung 1972, 22, 815) beziehungsweise können in analoger Weise hergestellt werden. Die Amine der allgemeinen Formel (III) und die Verbindungen der allgemeinen Formel (VI) sind Handelsprodukte.

Die Toxizität der Verbindungen der allgemeinen Formeln (I) und (II) ist gering, der LD₅₀-Wert (an Ratten und Mäusen p.o.) liegt im allgemeinen unter 250 mg/kg.

Die Verbindungen der allgemeinen Formel (I) und ihre physiologisch verträglichen Salze verfügen über eine bedeutende gastroprotektive Wirkung. Sie üben sowohl im Magen wie auch im Dünndarm eine Schutz- und Heilwirkung aus. Die antiulcerogene Wirkung der Verbindungen der allgemeinen Formel (I) wurde in Standard-Tests nachgewiesen. Als Beispiel ist in Tabelle 1 die auf Magengeschwüre ausgeübte Wirkung gezeigt, welche mittels 0,5 ml eines Gemisches von 96 %igem Alkohol und Salzsäure im Volumenverhältnis 1:0,02 an Ratten ausgelöst wurden (Gastroenterology 1979, 77, 433).

Tabelle 2 zeigt die Wirkung gegen Ulcus an Ratten, hervorgerufen durch Indomethacin (Arch. Int. Pharmacodyn. 1964, 117, 113).

**Tabelle 1**

| Die Schutzwirkung gegen durch salzsäurehaltigen 96 %igen Äthanol hervorgerufene Magenschleimhautschädigungen an Ratten | | |
|---|---|---|
| Name der Verbindung | Dosis mg/kg p.o. | Ulcushemmung % |
| N-tert.-Pentyl-6-methyl-4-oxo-4H-pyrido[1,2-a]pyrimidin-3-carboxamid | 50 | 77 |
| N-Isopropyl-6-methyl-4-oxo-4H-pyrido[1,2-a]pyrimidin-3-carboxamid | 50 | 95 |
| N-Neopentyl-6-methyl-4-oxo-4H-pyrido[1,2-a]pyrimidin-3-carboxamid | 100 | 48 |
| 6-Methyl-4-oxo-3-(pyrrolidinocarbonyl)-4H-pyrido[1,2-a]pyrimidin | 50 | 53 |
| N,6-Dimethyl-4-oxo-4H-pyrido[1,2-a]-pyrimidin-3-carboxamid | 100 | 48 |
| N-Propyl-6-methyl-4-oxo-4H-pyrido[1,2-a]pyrimidin-3-carboxamid | 12,5 | 69 |

**Tabelle 2**

| Hemmung der durch Indomethacin ausgelösten Magenschleimhautschädigung an Ratten | | |
|---|---|---|
| Name der Verbindung | Dosis mg/kg p.o. | Ulcushemmung % |
| 6-Methyl-4-oxo-3-(pyrrolidino)-carbonyl)-4H-pyrido[1,2-a]pyrimidin | 50 | 46 |
| N,6-Dimethyl-4-oxo-4H-pyrido[1,2-a]pyrimidin-3-carboxamid | 50 | 64 |
| N-tert.-Octyl-6-methyl-4-oxo-4H-pyrido[1,2-a]pyrimidin-3-carboxamid | 50 | 15 |
| N-Neopentyl-6-methyl-4-oxo-4H-pyrido[1,2-a]pyrimidin-3-carboxamid | 50 | 43 |
| N-tert.-Pentyl-6-methyl-4-oxo-4H-pyrido[1,2-a]pyrimidin-3-carboxamid | 100 | 38 |
| N-Isopropyl-6-methyl-4-oxo-4H-pyrido[1,2-a]pyrimidin-3-carboxamid | 100 | 88 |
| N-Propyl-6-methyl-4-oxo-4H-pyrido[1,2-a]pyrimidin-3-carboxamid | 12,5 | 48 |

Als Beispiel zu Hemmwirkung auf an Ratten durch Indomethacin hervorgerufene Darmgeschwüre sei die Wirkung der Verbindung N-Propyl-6-methyl-4-oxo-4H-pyrido[1,2-a]pyrimidin-3-carboxamid genannt. An vier aufeinanderfolgenden Tagen wurde die Verbindung in Dosen von 50 mg/kg verabreicht. Am zweiten Tag wurde den nicht ausgehungerten Ratten oral Indomethacin in einer Dosis von 15 mg/kg injiziert. Die Geschwürbildung im Dünndarm wurde am dritten Tag nach der Gabe des Indomethacins mit der Methode von Tsuromi (J. Pharm. Dyn. 1980, 3, 659) bestimmt. Bezogen auf die Kontrollgruppe hemmte die Verbindung die Geschwürbildung um 58 %.

Die Verbindungen der allgemeinen Formel (I) und ihre Salze können in der Heilkunde in Form von den Wirkstoff und inerte feste oder flüssige, organische oder anorganische Trägerstoffe enthaltenden Präparaten angewendet werden. Die Präparate werden auf die in der Arzneimittelindustrie übliche Weise hergestellt.

Es können Präparate zur oralen oder parenteralen Verabreichung, zum Beispiel Tabletten, Dragees, Kapseln oder deren retardierte Formen hergestellt werden. Die Präparate können geeignete feste Träger- und Streckmittel, steriles wässriges Lösungsmittel oder nicht-toxische organische Lösungsmittel enthalten. Den für die orale Verabreichung vorgesehen Präparaten können die für diesen Zweck üblichen Süß- und Geschmacksstoffe zugesetzt werden.

Oral einnehmbare Tabletten können als Trägerstoff zum Beispiel Lactose, Natriumcitrat, Kaliumcarbonat, ferner Sprengmittel (zum Beispiel Stärke, Alginsäure), Gleitmittel (zum Beispiel Talkum, Natriumlaurylsulfat, Magnesiumstearat) enthalten. Der Trägerstoff der Kapseln kann Lactose und Polyäthylenglykol sein. Die wässrigen Suspensionen können Emulgier- oder Suspendiermittel enthalten. Als Verdünnungsmittel der mit organischen Lösungsmitteln bereiteten Suspensionen kommen Äthanol, Glycerin, Chloroform usw. in Frage.

Die zur parenteralen Anwendung vorgesehenen Präparate sind Lösungen oder Suspensionen des Wirkstoffes in einem geeigneten Medium (zum Beispiel, Erdnußöl, Sesamöl, Polypropylenglykol oder Wasser).

Der Wirkstoffgehalt der Präparate kann innerhalb eines weiten Bereiches variieren und zwischen 0,005 und 99 % liegen.

Die tägliche Wirkstoffdosis kann innerhalb eines weiten Bereiches variiert werden und hängt vom Alter, vom Gewicht des Patienten, der Schwere seines Leidens der Zubereitungsform des Präparates und der Aktivität des jeweiligen Wirkstoffes ab. Im Falle oraler Verabreichung beträgt die Tagesdosis im allgemeinen 0,05-15 mg/kg als einmalige Gabe oder über mehrere Einzeldosen verteilt. Diese Angaben haben orientierenden Charakter, abhängend von den Bedingungen des jeweiligen Falles und den Vorschriften des Arztes kann die jeweils verabfolgte Menge geringer oder größer sein. In begründeten Fällen können auch andere Applikationsformen als die oben genannten gewählt werden.

Die Erfindung wird im folgenden an Hand von Beispielen näher erläutert, ist jedoch nicht auf diese Beispiele beschränkt.

### Beispiel 1

Ein Gemisch aus 16,32 g (0,08 Mol) 6-Methyl-4-oxo-4H-pyrido[1,2-a]pyrimidin-3-carbonsäure, 12,4 ml (0,088 Mol) Triäthylamin und 480 ml Chloroform wird von zu en mit einer Salz-Eis-Kältemischung unter Rühren gekühlt. Bei -20 °C wird dem Reaktionsgemisch innerhalb von 10 Minuten die Lösung von 6,5 ml (0,08 Mol) Chlorameisensäuremethylester in 40 ml Chloroform zugesetzt. Das Gemisch wird 5 Minuten lang gerührt und dann bei -18 °C innerhalb von 30 Minuten mit der Lösung von 2,5 g (0,08 Mol) Methylamin in 80 ml Chloroform (hergestellt aus Methylaminhydrochlorid mit Triäthylamin in Chloroform) versetzt. Das Gemisch wird noch eine Stunde lang bei unter -10 °C gerührt und dann über Nacht in den Kühlschrank gestellt. Anderntags wird das Gemisch zunächst dreimal mit je 140 ml 5 m/v-%iger (Masse pro Volumen, gemischte Prozente) Natriumhydrogencarbonatlösung und dann mit 140 ml Wasser ausgeschüttelt. Die organische Phase wird über Natriumsulfat getrocknet und unter vermindertem Druck eingedampft. Der Rückstand wird bei Raumtemperatur mit 150 ml 10 m/v-%iger Salzsäure verrührt. Nach einer Stunde wird die nicht umgesetzte 6-Methyl-4-oxo-4H-pyrido[1,2-a]pyrimidin-3-carbonsäure (10 g) abfiltriert. Der pH-Wert des wässrigen Filtrats wird mit 20 m/v-%iger Natronlauge auf 9 gestellt und das Gemisch dreimal mit je 100 ml Chloroform ausgeschüttelt. Die organischen Phasen werden vereinigt, über Natriumsulfat getrocknet und dann eingedampft. Der Rückstand wird aus Äthanol umkristallisiert. Man erhält 3,2 g (45,5 %) N,6-Dimethyl-4-oxo-4H-pyrido[1,2-a]pyrimidin-3-carboxamid. Das zitronengelbe Produkt schmilzt bei 208-210 °C.

| Analyse für C₁₁H₁₁N₃O₂ | | | |
|---|---|---|---|
| berechnet, %: | C 60,82 | H 5,10 | N 19,34 |
| gefunden, %: | C 60,91 | H 5,18 | N 19,31. |

### Beispiel 2

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit Salz-Eis-Kältemischung auf unter 10 °C gekühlt. Bei dieser Temperatur wird innerhalb von 20 Minuten tropfenweise die Lösung von 6,5 ml (0,08 Mol) Chlorameisensäuremethylester in 40 ml Chloroform zugegeben. Das Gemisch wird dann 5 Minuten lang gerührt und bei -15 °C innerhalb von 30 Minuten tropfenweise mit der Lösung von 5,2 g (0,088 Mol) Propylamin in 80 ml Chloroform versetzt. Das Gemisch wird bei unter -10 °C noch eine Stunde lang gerührt und bis zum nächsten Tag auf Eis gestellt. Zur Aufarbeitung wird das Gemisch zunächst mit 3x150 ml 5 %iger Natriumhydrogencarbonatlösung, dann mit 3x150 ml Wasser ausgeschüttelt. Die organische Phase wird über wasserfreiem Natriumsulfat getrocknet, filtriert und dann eingedampft. Man erhält 16,8 g (85,6 %) N-Propyl-6-methyl-4-oxo-4H-pyrido[1,2-a]pyrimidin-3-carbonsäureamid, das nach Umkristallisieren aus Äthanol als gelbe kristalline Substanz vorliegt und bei 158-160 °C schmilzt.

| Analyse für C₁₃H₁₅N₃O₂ | | | |
|---|---|---|---|
| berechnet, %: | C 63,66 | H 6,16 | N 17,13 |
| gefunden, %: | C 63,72 | H 6,24 | N 17,34. |

### Beispiel 3

Unter Rühren wird bie Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und dann mit einer Salz-Bis-Kältemischung auf unter -10 °C gekühlt. Bei Temperaturen zwischen -12 und -16 °C wird innerhalb von 30 Minuten tropfenweise die Lösung von 6,5 ml (0,08 Mol) Chlorameisensäuremethylester in 40 ml Chloroform zugegeben. Das Gemisch wird 5 Minuten lang gerührt und dann bei -15 °C innerhalb von 40 Minuten tropfenweise mit der Lösung von 5,2 g (0,088 Mol) Isopropylamin in 80 ml Chloroform versetzt. Das Gemisch wird bei der genannten Temperatur noch eine Stunde lang gerührt und dann auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet, filtriert und dann eingedampft. Man erhält 13,2 g (67,3 %) N-Isopropyl-6-methyl-4-oxo-4H-pyrido[1,2-a]pyrimidin-3-carbonsäureamid, das nach Umkristallisieren aus Äthanol als gelbe kristalline Substanz vorliegt und bei 165-169 °C schmilzt.

| Analyse für C₁₃H₁₅N₃O₂ | | | |
|---|---|---|---|
| berechnet, %: | C 63,66 | H 6,16 | N 17,13 |
| gefunden, %: | C 63,57 | H 6,09 | N 17,18. |

### Beispiel 4

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Bis-Kältemischung auf unter -10 °C gekühlt. Bei Temperaturen zwischen -12 und -15 °C wird innerhalb von 15 Minuten tropfenweise die Lösung von 6,5 ml (0,08 Mol) Chlorameisensäuremethylester in 40 ml Chloroform zugegeben. Das Gemisch wird noch 5 Minuten lang gerührt und dann bei einer Temperatur zwischen -10 und -15 °C innerhalb von 50 Minuten tropfenweise mit der Lösung von 6,43 g (0,088 Mol) Butylamin in 80 ml Chloroform versetzt. Das Gemisch wird bei der genannten Temperatur noch eine Stunde lang gerührt und dann auf Eis gestellt. Zur Aufarbeitung wird das Gemisch zuerst mit 3x150 ml 5 %-iger Natriumhydrogencarbonatlösung, dann mit 3x150 ml Wasder ausgeschüttelt. Die organische Phase wird über wasserfreiem Natriumsulfat getrocknet und dann eingedampft. Man erhält 14,9 g (71,8 %) N-Butyl-6-methyl-4-oxo-4H-pyrido[1,2-a]pyrimidin-3-carbonsäureamid in Form gelber Kristalle, die bei 127-130 °C schmelzen.

| Analyse für C₁₄H₁₇N₃O₂ | | | |
|---|---|---|---|
| berechnet, %: | C 64,85 | H 6,61 | N 16,20 |
| gefunden, %: | C 64,78 | H 6,54 | N 16,27 |

### Beispiel 5

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit Salz-Bis-Kältemischung auf unter -10 °C gekühlt. Bei -20 °C wird innerhalb von 20 Minuten tropfenweise die Lösung von 6,5 ml (0,08 Mol) Chlorameisensäuremethylester in 40 ml Chloroform zugegeben. Das Gemisch wird noch 5 Minuten lang gerührt und dann bei -18 bis -20 °C innerhalb von 50 Minuten tropfenweise mit der Lösung von 6,47 g (0,088 Mol) tert.-Butylamin in 80 ml Chloroform versetzt. Das Gemisch wird bei der genannten Temperatur noch eine Stunde lang gerührt und dann über Nacht in den Kühlschrank gestellt. Zur Aufarbeitung wird das Gemisch zunächst mit 3x150 ml 5 %iger Natriumhydrogencarbonatlösung, dann mit 3x150 ml Wasser ausgeschüttelt. Die organische Phase wird über wasserfreiem Natriumsulfat getrocknet und dann eingedampft. Man erhält 15,5 g (74,7 %) N-tert.-Butyl-6-methyl-4-oxo-4H-pyrido[1,2-a]pyrimidin-3-carbonsäureamid, das nach Umkristallisieren aus Äthanol als blaß sandfarbene Substanz vorliegt und bei 164-166 °C schmilzt.

| Analyse für C₁₄H₁₇N₃O₂ | | | |
|---|---|---|---|
| berechnet, %: | C 64,85 | H 6,61 | N 16,20 |
| gefunden, %: | C 64,91 | H 6,75 | N 16,23. |

### Beispiel 6

Unter Rühren wird bei Raumtemperatur ein Gemisch aus 8,16 g (0,04 Mol) 6-Methyl-4-oxo-4H-pyrido[1,2-a]pyrimidin-3-carbonsäure, 6,2 ml (0,044 Mol) Triäthylamin und 240 ml Chloroform bereitet und mit einer Salz-Eis-Kältemischung auf unter -10 °C gekühlt. Bei -15 °C wird innerhalb von 20 Minuten tropfenweise die Lösung von 4 ml (0,042 Mol) Chlorameisensäureäthylester in 20 ml Chloroform zugegeben. Das Gemisch wird noch 5 Minuten lang gerührt und dann bei -15 °C innerhalb von 20 Minuten tropfenweise mit der Lösung von 3,835 g (0,044 Mol) n-Pentylamin in 40 ml Chloroform versetzt. Das Gemisch wird bei der genannten Temperatur noch eine Stunde lang gerührt und dann über Nacht auf Eis gestellt. Zur Aufarbeitung wird das Gemisch zuerst mit 3x100 ml 5 %iger Natriumhydrogencarbonatlösung, dann mit 3x100 ml Wasser ausgeschüttelt. Die organische Phase wird über wasserfreiem Natriumsulfat getrockent und dann eingedampft. Man erhält 6,1 g (55,8 %) N-Pentyl-6-methyl-4-oxo-4H-pyrido[1,2-a]pyrimidin-3-carboxamid, das nach Umkristallisieren aus Äthanol als orangefarbene kristalline Substanz vorliegt, die bei 112-116 °C schmilzt.

| Analyse für C₁₅H₁₉N₃O₂ | | | |
|---|---|---|---|
| berechnet, %: | C 65,91 | H 7,01 | N 15,37 |
| gefunden, %: | C 65,87 | H 7,13 | N 15,45. |

### Beispiel 7

Unter Rühren wird bei Raumtemperatur ein Gemisch aus 8,16 g (0,04 Mol) 6-Methyl-4-oxo-4H-pyrido[1,2-a]pyrimidin-3-carbonsäure, 6,2 ml (0,044 Mol) Triäthylamin und 240 ml Chloroform bereitet und mit einer Salz-Eis-Kältemischung auf unter -10 °C gekühlt. Bei -16 °C wird innerhalb von 10 Minuten tropfenweise die Lösung von 3,25 ml (0,04 Mol) Chlorameisensäuremethylester in 20 ml Chloroform zugegeben. Das Gemisch wird noch 5 Minuten lang gerührt und dann bei -10 °C innerhalb von 5 Minuten tropfenweise mit der Lösung von 3,83 g (0,044 Mol) Neopentylamin in 40 ml Chloroform versetzt. Das Gemisch wird bei unter -10 °C noch eine Stunde lang gerührt und dann zuerst mit 3x80 ml 5 %iger Natriumhydrogencarbonatlösung, dann mit 3x80 ml Wasser ausgeschüttelt. Die organische Phase wird über wasserfreiem Natriumsulfat getrocknet und dann eingedampft. Man erhält 6,8 g (62,2 %) N-Neopentyl-6-methyl-4-oxo-4H-pyrido[1,2-a]pyrimidin-3-carboxamid, das nach Umkristallisieren aus Äthanol als kristalline gelbe Substanz vorliegt und bei 191-194 °C schmilzt.

| Analyse für C₁₅H₁₉N₃O₂ | | | |
|---|---|---|---|
| berechnet, %: | C 65,91 | H 7,01 | N 15,37 |
| gefunden, %: | C 65,89 | H 6,95 | N 15,39. |

### Beispiel 8

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt.Bei -11 bis -12 °C wird innerhalb von 20 Minuten tropfenweise die Lösung von 6,5 g (0,08 Mol) Chlorameisensäuremethylester in 40 ml Chloroform zugegeben. Das Gemisch wird 5 Minuten lang gerührt und dann bei -12 °C innerhalb von 35 Minuten tropfenweise mit der Lösung von 8,9 g (0,088 Mol) Hexylamin in 80 ml abs. Chloroform versetzt. Das Gemisch wird bei der genannten Temperatur noch eine Stunde lang gerührt und dann über Nacht auf Eis gestellt. Zur Aufarbeitung wird das Gemisch zuerst mit 3x150 ml 5 %-iger Natriumhydrogencarbonatlösung, dann mit 3x150 ml Wasser ausgeschüttelt. Die organische Phase wird über wasserfreiem Natriumsulfat getrocknet, filtriert und dann eingedampft. Man erhält 16,4 g (71,3 %) N-Hexyl-6-methyl-4-oxo-4H-pyrido[1,2-a]pyrimidin-3-carbonsäureamid, das nach Umkristallisieren aus Äthanol als gelbe kristalline Substanz vorliegt, die bei 116-118 °C schmilzt.

| Analyse für C₁₆H₂₁N₃O₂ | | | |
|---|---|---|---|
| berechnet, %: | C 66,87 | H 7,36 | N 14,62 |
| gefunden, %: | C 66,77 | H 7,28 | N 14,68. |

### Beispiel 9

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -12 bis -15 °C wird innerhalb von 20 Minuten tropfenweise die Lösung von 6,5 ml (0,08 Mol) Chloroameisensäuremethylester in 40 ml Chloroform zugegeben. Das Gemisch wird 5 Minuten lang gerührt und dann bei -11 bis -16 °C innerhalb von 50 Minuten tropfenweise mit der Lösung von 10,14 g (0,088 Mol) Heptylamin in 80 ml Chloroform versetzt. Das Gemisch wird bei der genannten Temperatur noch eine Stunde lang gerührt und dann auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet und dann eingedampft. Man erhält 17,0 g (70,5 %) N-Heptyl-6-methyl-4-oxo-4H-pyrido[1,2-a]pyrimidin-3-carbonsäureamid in Form organgefarbener Kristalle, die bei 118-120 °C schmelzen.

| Analyse für C₁₇H₂₃N₃O₂ | | | |
|---|---|---|---|
| berechnet, %: | C 67,75 | H 7,69 | N 13,94 |
| gefunden, %: | C 67,87 | H 7,74 | N 13,89. |

### Beispiel 10

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -13 bis -16 °C wird innerhalb von 25 Minuten tropfenweise die Lösung von 6,5 ml (0,08 Mol) Chlorameisensäuremethylester in 40 ml Chloroform zugegeben Das Gemisch wird noch 5 Minuten lang gerührt und dann bei -13 bis -16 °C innerhalb von 40 Minuten tropfenweise mit der Lösung von 11,37 g (0,088 Mol) Octylamin in 80 ml Chloroform versetzt. Das Gemisch wird bei der genannten Temperatur noch eine Stunde lang gerührt und dann über Nacht auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet und dann eingedampft. Man erhält 15,4 g (61,0 %) N-Octyl-6-methyl-4-oxo-4H-pyrido[1,2-a]pyrimidin-3-carbonsäureamid, das nach Umkristallisieren aus Äthanol als hellgelbe kristalline Substanz vorliegt, die bei 126-129 °C schmilzt.

| Analyse für C₁₈H₂₅N₃O₂ | | | |
|---|---|---|---|
| berechnet, %: | C 68,54 | H 7,99 | N 13,32 |
| gefunden, %: | C 68,68 | H 8,04 | N 13,40. |

### Beispiel 12

Unter Rühren wird bei Raumtemperatur ein Gemisch aus 8,16 g (0,04 Mol) 6-Methyl-4-oxo-4H-pyrido[1,2-a]pyrimidin-3-carbonsäure, 240 ml Chloroform und 6,2 ml (0,044 Mol) Triäthylamin bereitet und mit einer Salz-Eis-Kältemischung auf unter -10 °C gekühlt. Innerhalb von 15 Minuten wird bei -12 bis -17 °C tropfenweise die Lösung von 4 ml (0,042 Mol) Chlorameisensäureäthylester in 20 ml Chloroform zugegeben. Das Gemisch wird noch 5 Minuten lang gerührt und dann bei der gleichen Temperatur innerhalb von 15 Minuten tropfenweise mit der Lösung von 3,84 g (0,044 Mol) tert.-Pentylamin in 40 ml Chloroform versetzt. Das Gemisch wird bei etwa -10 °C noch eine Stunde lang gerührt und dann über Nacht auf Eis gestellt. Zur Aufarbeitung wird das Gemisch zuerst mit 3x50 ml 5 %iger Natriumhydrogencarbonatlösung, dann mit 3x50 ml Wasser ausgeschüttelt. Die organische Phase wird über wasserfreiem Natriumsulfat getrocknet und nach dem Filtrieren eingedampft. Man erhält 5,9 g (54 %) langsam kristallisierendes N-tert.-Pentyl-6-methyl-4-oxo-4H-pyrido[1,2-a]pyrimidin-3-carboxamid, das nach Umkristallisieren aus Äthanol bei 87-89 °C schmilzt.

| Analyse für C₁₅H₁₉N₃O₂ | | | |
|---|---|---|---|
| berechnet, %: | C 65,90 | H 7,00 | N 15,37 |
| gefunden, %: | C 65,97 | H 7,11 | N 15,47. |

### Beispiel 13

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -10 bis -17 °C wird innerhalb von 25 Minuten tropfenweise die Lösung von 6,5 ml Chlorameisensäuremethylester in 40 ml Chloroform zugegeben. Das Gemisch wird 5 Minuten lang gerührt und dann bei der gleichen Temperatur innerhalb von 40 Minuten tropfenweise mit der Lösung von 5,025 g (0,088 Mol) Cyclopropylamin in 80 ml Chloroform versetzt. Das Gemisch wird bei unter -10 °C noch eine Stunde lang gerührt und dann über Nacht auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet und nach dem Filtrieren eingedampft. Man erhält 14,5 g (74,5 %) N-Cyclopropyl-6-methyl-4-oxo-4H-pyrido[1,2-a]pyrimidin-3-carbonsäureamid, das nach Umkristallisieren aus Äthanol als blaßgelbe kristalline Substanz vorliegt, die bei 174-176 °C schmilzt.

| Analyse für C₁₃H₁₃N₃O₂ | | | |
|---|---|---|---|
| berechnet, %: | C 64,18 | H 5,38 | N 17,27 |
| gefunden, %: | C 64,25 | H 5,42 | N 17,21. |

### Beispiel 14

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -10 °C wird innerhalb von 20 Minuten tropfenweise die Lösung von 6,5 ml (0,08 Mol) Chlorameisensäuremethylester in 40 ml Chloroform zugegeben. Das Gemisch wird noch 5 Minuten gerührt und dann bei -15 °C innerhalb von 30 Minuten tropfenweise mit der Lösung von 7,49 g (0,088 Mol) Cyclopentylamin in 80 ml Chloroform versetzt. Das Gemisch wird bei etwa -10 °C noch eine Stunde lang gerührt und dann über Nacht auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet und nach dem Filtrieren eingedampft. Man erhält 14,3 g (65,9 %) N-Cyclopentyl-6-methyl-4-oxo-4H-pyrido[1,2-a]pyrimidin-3-carbonsäureamid, das nach Umkristallisieren aus Äthanol als gelbe kristalline Substanz vorliegt, die bei 158-160 °C schmilzt.

| Analyse für C₁₅H₁₇N₃O₂ | | | |
|---|---|---|---|
| Berechnet, %: | C 66,04 | H 6,31 | N 15,49 |
| gefunden, %: | C 66,34 | H 6,28 | N 15,54. |

### Beispiel 15

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -10 °C wird innerhalb von 20 Minuten tropfenweise die Lösung von 6,5 ml (0,08 Mol) Chlorameisensäuremethylester in 40 ml Chloroform zugegeben. Das Gemisch wird 5 Minuten lang gerührt und dann bei -15 °C innerhalb von 30 Minuten tropfenweise mit der Lösung von 8,73 g (0,088 Mol) Cyclohexylamin in 80 ml Chloroform versetzt. Das Gemisch wird bei etwa - 10 °C eine Stunde lang gerührt und dann über Nacht auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet und nach dem Filtrieren eingedampft. Man erhält 16,7 g (73,2 %) N-Cyclohexyl-6-methyl-4-oxo-4H-pyrido[1,2-a]pyrimidin-3-carbonsäureamid, das nach Umkristallisieren aus Äthanol als gelbe kristalline Substanz vorliegt und bei 174-176 °C schmilzt.

| Analyse für C₁₆H₁₉N₃O₂ | | | |
|---|---|---|---|
| berechnet, %: | C 67,35 | H 6,71 | N 14,72 |
| gefunden, %: | C 67,29 | H 6,67 | N 14,78 |

### Beispiel 16

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -10 °C wird innerhalb von 20 Minuten tropfenweise die Lösung von 6,5 ml (0,08 Mol) Chlorameisensäuremethylester in 40 ml Chloroform gegeben. Das Gemisch wird 5 Minuten lang gerührt und dann bei -12 bis -15 °C innerhalb von 40 Minuten tropfenweise mit der Lösung von 9,96 g (0,088 Mol) Cycloheptylamin in 80 ml Chloroform versetzt. Das Reaktionsgemisch wird bei etwa -10 °C noch eine Stunde lang gerührt und dann über Nacht auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet und nach dem Filtrieren eingedampft. Man erhält 20,4 g (85,2 %) N-Cycloheptyl-6-methyl-4-oxo-4H-pyrido[1,2-a]pyrimidin-3-carbonsäureamid in Form einer kristallinen Substanz, die bei 172-174 °C schmilzt.

| Analyse für C₁₇H₂₁N₃O₂ | | | |
|---|---|---|---|
| berechnet, %: | C 68,20 | H 7,07 | N 14,03 |
| gefunden, %: | C 68,27 | H 7,18 | N 14,10. |

### Beispiel 17

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -10 bis -13 °C wird innerhalb von 20 Minuten tropfenweise die Lösung von 6,5 ml (0,08 Mol) Chlorameisensäuremethylester in 40 ml Chloroform zugegeben. Das Gemisch wird 5 Minuten lang gerührt und dann bei -10 bis -13 °C innerhalb von 30 Minuten tropfenweise mit der Lösung von 9,19 g (0,088 Mol) Anilin in 80 ml Chloroform versetzt. Das Gemisch wird bei etwa -10 °C noch eine Stunde lang gerührt und dann über Nacht auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet und nach dem Filtrieren eingedampft. Man erhält 17,6 (78,8 %) N-Phenyl-6-methyl-4-oxo-4H-pyrido[1,2-a]pyrimidin-3-carbonsäureamid, das nach Umkristallisieren aus Äthanol als gelbe kristalline Substanz vorliegt, die bi 186-188 °C schmilzt.

| Analyse für C₁₆H₁₃N₃O₂ | | | |
|---|---|---|---|
| berechnet, %: | C 68,81 | H 4,69 | N 15,04 |
| gefunden, %: | C 68,87 | H 4,78 | N 15,10. |

### Beispiel 18

Unter Rühren wird bei Raumtemperatur ein Gemisch aus 16,32 g (0,08Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -12 bis -17 °C wird innerhalb von 25 Minuten tropfenweise die Lösung von 6,5 ml (0,08 Mol) Chlorameisensäuremethylester in 40 ml Chloroform zugegeben. Das Gemisch wird 5 Minuten lang gerührt und dann bei -13 °C innerhalb von 30 Minuten tropfenweise mit der Lösung von 9,78 g (0,088 Mol) p-Fluoranilin in 80 ml Chloroform versetzt. Das Gemisch wird bei etwa -10 °C eine Stunde lang gerührt und dann über Nacht auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet und nach dem Filtrieren eingedampft. Man erhält 18,3 g (77 %) N-(p-Fluorphenyl)-6-methyl-4-oxo-4H-pyrido[1,2-a]pyrimidin-3-carbonsäureamid, das nach Umkristallisieren aus n-Propanol gelbe Kristalle bildet und bei 202-204 °C schmilzt.

| Analyse für C₁₆H₁₂N₃O₂F | | | |
|---|---|---|---|
| berechnet, %: | C 64,64 | H 4,07 | N 14,13 |
| gefunden, %: | C 64,58 | H 4,11 | N 14,18 |

### Beispiel 19

Unter Rühren wird bei Raumtemperatur ein Gemisch aus 16,32 g (0,08 Mol) 6-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -10 bis -12 °C wird innerhalb von 20 Minuten tropfenweise die Lösung von 6,5 ml (0,08 Mol) Chlorameisensäuremethylester in 40 ml Chloroform zugesetzt. Das Gemisch wird 5 Minuten lang gerührt und dann bei -10 °C innerhalb von 35 Minuten tropfenweise mit der Lösung von 11,23 g (0,088 Mol) p-Chloranilin in 80 ml Chloroform versetzt. Das Gemisch wird bei etwa -10 °C noch eine Stunde lang gerührt und dann über Nacht auf Eis gestellt. Zur Aufarbeitung wird das Gemisch zuerst mit 3x150 ml 5 %iger Natriumhydrogencarbonatlösung, dann mit 3x150 ml Wasser ausgeschüttelt. Die organische Phase wird über wasserfreiem Natriumsulfat getrocknet und nach dem Filtrieren eingedampft. Man erhält 18,5 (73,7 %) N-(p-Chlorphenyl)-6-methyl-4-oxo-4H-pyrido[1,2-a]pyrimidin-3-carbonsäureamid, das nach Umkristallisieren aus Äthanol gelbe Kristalle bildet, die bei 202-204 °C schmelzen.

| Analyse für C₁₆H₁₂N₃O₂Cl | | | |
|---|---|---|---|
| berechnet, %: | C 61,25 | H 3,85 | N 13,39 |
| gefunden, %: | C 61,36 | H 3,90 | N 13,42. |

### Beispiel 20

Unter Rühren wird bei Raumtemperatur ein Gemisch aus 7,2 g (0,0378 Mol) 4-Oxo-4H-pyrido[1,2-a]pyrimidin-3-carbonsäure, 240 ml Chloroform und 6,2 g (0,044 Mol) Triäthylamin bereitet und mit einer Salz-Eis-Kältemischung auf unter -10 °C gekühlt. Bei -20 °C wird innerhalb von 20 Minuten tropfenweise die Lösung von 3,25 ml (0,044 Mol) Chlorameisensäuremethylester in 20 ml Chloroform zugegeben. Das Gemisch wird 5 Minuten lang gerührt und dann bei -18 °C innerhalb einer Stunde tropfenweise mit der Lösung von 2,6 g (0,04 Mol) n-Propylamin in 80 ml Chloroform versetzt. Das Gemisch wird bei der genannten Temperatur noch eine Stunde lang gerührt und dann über Nacht auf Eis gestellt. Zur Aufarbeitung wird das Gemisch zuerst mit 3x70 ml 5 %iger Natriumhydrogencarbonatlösung, dann mit 3x70 ml Wasser ausgeschüttelt. Die organische Phase wird über wasserfreiem Natriumsulfat getrocknet und nach dem Filtrieren eingedampft. Man erhält 7,1 g (77,5 %) N-Propyl-4-oxo-4H-pyrido[1,2-a]pyrimidin-3-carbonsäureamid, das nach Umkristallisieren aus Äthanol blaßgelbe Kristalle bildet, die bei 120-122 °C schmelzen.

| Analyse für C₁₂H₁₁N₃O₂ | | | |
|---|---|---|---|
| berechnet, %: | C 62,87 | H 4,83 | N 18,33 |
| gefunden, %: | C 62,81 | H 4,79 | N 18,39. |

### Beispiel 21

Unter Rühren wird bei Raumtemperatur ein Gemisch aus 16,32 g (0,08 Mol) 7-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -18 °C wird innerhalb von 20 Minuten tropfenweise die Lösung von 6,5 ml (0,08 Mol) Chloroameisensäuremethylester in 40 ml Chloroform zugegeben. Das Gemisch wird 5 Minuten lang gerührt und dann bei -18 °C innerhalb einer Stunde tropfenweise mit der Lösung von 5,2 g (0,088 Mol) Propylamin in 80 ml Chloroform versetzt. Das Gemisch wird bei -10 °C noch eine Stunde lang gerührt und dann über Nacht auf Eis gestellt. Zur Aufarbeitung wird das Gemisch zuerst mit 3x150 ml 5 m/v-%iger Natriumhydrogencarbonatlösung, dann mit 3x150 ml Wasser ausgeschüttelt. Die organische Phase wird über wasserfreiem Natriumsulfat getrocknet und nach dem Filtrieren eingedampft. Man erhält 17,5 g (89,2 %) N-Propyl-7-methyl-4-oxo-4H-pyrido[1,2-a]pyrimidin-3-carbonsäureamid, das nach Umkristallisieren aus Äthanol eine bei 126-128 °C schmelzende kristalline Substanz darstellt.

| Analyse für C₁₃H₁₅N₃O₂ | | | |
|---|---|---|---|
| berechnet, %: | C 63,66 | H 6,16 | N 17,13 |
| gefunden, %: | C 63,69 | H 6,21 | N 17,18. |

### Beispiel 22

Unter Rühren wird bei Raumtemperatur ein Gemisch aus 16,32 g (0,08 Mol) 8-Methyl-4-oxo-4H-pyrido[1,2-a]pyrimidin-3-carbonsäure, 480 ml Chloroform und 12,4 ml (0,088 Mol) Triäthylamin bereitet und mit einer Salz-Eis-Kältemischung auf unter -10 °C gekühlt. Bei -18 °C wird innerhalb von 10 Minuten tropfenweise die Lösung von 6,5 ml (0,08 Mol) Chlorameisensäuremethylester in 40 ml Chloroform zugegeben. Das Gemisch wird 5 Minuten lang gerührt und dann bei -18 °C innerhalb einer Stunde tropfenweise mit der Lösung von 5,2 g (0,088 Mol) Propylamin in 80 ml Chloroform versetzt. Das Gemisch wird bei -10 °C eine Stunde lang gerührt und dann über Nacht auf Eis gestellt. Zur Aufarbeitung wird das Gemisch zuerst mit 3x150 ml 5 m/v-%iger Natriumhydrogencarbonatlösung, dann mit 3x150 ml Wasser ausgeschüttelt. Die organische Phase wird über wasserfreiem Natriumsulfat getrocknet und nach dem Filtrieren eingedampft. Man erhält 17,3 g (88,2 %) N-Propyl-8-methyl-4-oxo-4H-pyrido[1,2-a]pyrimidin-3-carbonsäureamid, das nach Umkristallisieren aus Äthanol eine bei 194-196 °C schmelzende kristalline Substanz bildet.

| Analyse für C₁₃H₁₅N₃O₂ | | | |
|---|---|---|---|
| berechnet, %: | C 63,66 | H 6,16 | N 17,13 |
| gefunden, %: | C 63,58 | H 6,21 | N 17,19. |

### Beispiel 23

Unter Rühren wird bei Raumtemperatur ein Gemisch aus 9,2 g (0,045 Mol) 9-Methyl-4-oxo-4H-pyrido[1,2-a]pyrimidin-3-carbonsäure, 280 ml Chloroform und 7,05 ml (0,05 Mol) Triäthylamin bereitet und mit einer Salz-Eis-Kältemischung auf unter -10 °C gekühlt. Bei -21 °C wird innerhalb von 10 Minuten tropfenweise die Lösung von 3,7 ml (0,045 Mol) Chlorameisensäuremethylester in 25 ml Chloroform zugesetzt. Das Gemisch wird 5 Minuten lang gerührt und dann bei -19 bis -21 °C innerhalb von 45 Minuten tropfenweise mit der Lösung von 2,95 g (0,05 Mol) Propylamin in 50 ml Chloroform versetzt. Das Gemisch wird bei etwa -10 °C noch eine Stunde lang gerührt und dann über Nacht auf Eis gestellt. Zur Aufarbeitung wird das Gemisch zuerst mit 3x70 ml 5 m/v-%iger Natriumhydrogencarbonatlösung, dann mit 3x70 ml Wasser ausgeschüttelt. Die organische Phase wird über wasserfreiem Natriumsulfat getrocknet und nach dem Filtrieren eingedampft. Man erhält 7,8 g (70,7 %) N-Propyl-9-methyl-4-oxo-4H-pyrido[1,2-a]pyrimidin-3-carbonsäureamid, das nach Umdristallisieren aus Äthanol als schneeweiße, kristalline, bei 138-140 °C schmelzende Substanz vorliegt.

| Analyse für C₁₃H₁₅N₃O₂ | | | |
|---|---|---|---|
| berechnet, %: | C 63,66 | H 6,16 | N 17,13 |
| gefunden, %: | C 63,69 | H 6,21 | N 17,18. |

### Beispiel 24

Unter Rühren wird bei Raumtemperatur ein Gemisch aus 2,18 g (0,01 Mol) 6-Methyl-4-oxo-4H-pyrido[1,2-a]pyrimidin-3-essigsäure, 60 ml Chloroform und 1,55 ml (0,011 Mol) Triäthylamin bereitet und mit einer Salz-Eis-Kältemischung auf unter -10 °C gekühlt. Bei -12 °C wird innerhalb von 7 Minuten tropfenweise die Lösung von 0,945 g (0,01 Mol) Chlorameisensäuremethylester in 5 ml Chloroform zugegeben. Das Gemisch wird 5 Minuten lang gerührt und dann bei -15 °C innerhalb von 10 Minuten tropfenweise mit der Lösung von 0,65 g (0,011 Mol) Propylamin in 10 ml Chloroform versetzt. Das Gemisch wird bei dieser Temperatur noch eine Stunde lang gerührt und dann über Nacht auf Eis gestellt. Zur Aufarbeitung wird das Gemisch zuerst mit 3x40 ml 5 m/v-%iger Natriumhydrogencarbonatlösung, dann mit 3x40 ml Wasser ausgeschüttelt. Die organische Phase wird über wasserfreiem Natriumsulfat getrocknet und nach dem Filtrieren eingedampft. Der Rückstand wird mit kaltem Äthylacetat verrieben. Man erhält 0,8 g (30,9 %) N-Propyl-6-methyl-4-oxo-4H-pyrido[1,2-a]pyrimidin-3-essigsäureamid, das nach Umkristallisieren aus Äthylacetat eine bei 96-98 °C schmelzende kristalline Substanz darstellt.

| Analyse für C₁₄H₁₇N₃O₂ | | | |
|---|---|---|---|
| berechnet, %: | C 64,85 | H 6,61 | N 16,20 |
| gefunden, %: | C 64,81 | H 6,72 | N 16,28. |

### Beispiel 25

Unter Rühren wird bei Raumtemperatur ein Gemisch aus 5,717 g (0,028 Mol) 6-Methyl-4-oxo-4H-pyrido[1,2-a]pyrimidin-3-carbonsäure, 120 ml Chloroform und 4,34 ml (0,031 Mol) Triäthylamin bereitet und mit einer Salz-Eis-Kältemischung auf unter -10 °C gekühlt. Bei -10 °C wird innerhalb von 10 Minuten tropfenweise die Lösung von 2,52 ml (0,026 Mol) Chlorameisensäuremethylester in 10 ml Chloroform zugegeben. Das Gemisch wird 5 Minuten lang gerührt und dann bei -12 °C innerhalb von 30 Minuten tropfenweise mit der Lösung von 3,7 g (0,028 Mol) 2-Aminoisobuttersäureäthylester in 20 ml Chloroform versetzt. Das Gemisch wird bei etwa -10 °C noch eine Stunde lang gerührt und dann über Nacht auf Eis gestellt. Zur Aufarbeitung wird des Gemisch zuerst mit 3x50 ml 5 m/v-%iger Natriumhydrogencarbonatlösung, dann mit 3x50 ml Wasser ausgeschüttelt. Die organische Phase wird über wasserfreiem Natriumsulfat getrocknet und nach dem Filtrieren eingedampft. Man erhält 5,7 g (64,2 %) N-(1-Äthoxycarbonyl-1-methyl-äthyl)-6-methyl-4-oxo-4H-pyrido[1,2-a]pyrimidin-3-carbonsäureamid, das nach Umkristallisieren aus Äthanol gelbe Kristalle bildet, die bei 136 °C schmelzen.

| Analyse für C₁₆H₁₉N₃O₄ | | | |
|---|---|---|---|
| berechnet, %: | C 60,56 | H 6,03 | N 13,24 |
| gefunden, %: | C 60,63 | H 6,13 | N 13,27. |

### Beispiel 26

Unter Rühren wird bei Raumtemperatur ein Gemisch aus 4,08 g (0,02 Mol) 6-Methyl-4-oxo-4H-pyrido 1,2-a pyrimidin-3-carbonsäure, 120 ml Chloroform und 3,1 ml (0,022 Mol) Triäthylamin bereitet und mit einer Salz-Eis-Kältemischung auf unter -10 °C gekühlt. Bei -15 °C wird innerhalb von 10 Minuten tropfenweise die Lösung von 2,52 ml (0,026 Mol) Chlorameisensäuremethylester in 10 ml Chloroform zugegeben. Das Gemisch wird 5 Minuten lang gerührt und dan innerhalb von 15 Minuten mit der Lösung von 3,33 g (0,022 Mol) 1-Adamanthanamin in 20 ml Chloroform versetzt. wobei die Temperatur -15 °C beträgt. Das Gemisch wird bei etwa -10 °C eine Stunde lang gerührt und dann über Nacht auf Eis gestellt. Zur Aufarbeitung wird das Gemisch zuerst mit 3x70 ml 5 m/v-%iger Natriumhydrogencarbonatlösung, dann mit 3x70 ml Wasser ausgeschüttelt. Die organische Phase wird über wasserfreiem Natriumsulfat getrocknet und nach dem Filtrieren eingedampft. Man erhält 4,4 g (65,2 %) N-Adamantyl-6-methyl-4-oxo-4H-pyrido[1,2-a]pyrimidin-3-carbonsäureamid, das nach Umkristallisieren aus Äthanol hellgelbe Kristalle bildet, die bei 190-193 °C schmelzen.

| Analyse für C₂₀H₂₃N₃O₂ | | | |
|---|---|---|---|
| berechnet, %: | C 71,19 | H 6,87 | N 12,45 |
| gefunden, %: | C 71,24 | H 6,97 | N 12,51. |

### Beispiel 27

Unter Rühren wird bei Raumtemperatur ein Gemisch aus 20,4 g (0,1 Mol) 6-Methyl-4-oxo-4H-pyrido[1,2-a]pyrimidin-3-carbonsäure, 600 ml Chloroform und 15,5 ml (0,11 Mol) Triäthylamin bereitet und mit einer Salz-Eis-Kältemischung auf unter -10 °C gekühlt. Bei -10 °C wird innerhalb von 30-35 Minuten tropfenweise die Lösung von 10 ml (0,1 Mol) Chlorameisensäuremethylester in 50 ml Chloroform zugegeben. Dad Gemisch wird 20 Minuten lang gerührt und dann bei -5 bis -8 °C innerhalb einer Stunde tropfenweise mit dem Gemisch von 9 g (0,11 Mol) Pyrrolidin und 100 ml Chloroform versetzt. Das Gemisch wird bei der genannten Temperatur noch eine Stunde lang gerührt und dann über Nacht auf Eis gestellt. Zur Aufarbeitung wird des Gemisch zuerst mit 3x400 5 m/v-%iger Natriumhydrogencarbonatlösung, dann mit 3x400 ml Wasser ausgeschüttelt. Die organische Phase wird über wasserfreiem Natriumsulfat getrocknet und nach dem Filtrieren eingedampft. Der Rückstand wird in Petroläther suspendiert. Man erhält 8,3 g (32,3 %) 6-Methyl-4-oxo-4H-pyrido[1,2-a]pyrimidin-3-carbonsäure-pyrrolidin, das nach Umkristallisieren aus Äthanol eine bei 132-134 °C schmelzende gelbe Substanz bildet.

| Analyse für C₁₄H₁₅N₃O₂ | | | |
|---|---|---|---|
| berechnet, %: | C 65,36 | H 5,88 | N 16,33 |
| gefunden, %: | C 65,01 | H 6,04 | N 16,57. |

### Beispiel 28

1,527 g (0,00623 Mol) N-Propyl-6-methyl-4-oxo-4H-pyrido[1,2-a]pyrimidin-3-carbonsäureamid werden unter Erwärmen gelöst. Zu der klaren gelben Lösung werden tropfenweise 0,61 g (0,009 Mol) Methansulfonsäure gegeben. Beim Kühlen der Lösung fallen 1,84 g (86,8 %) einer weißen, kristallinen Substanz aus. Die erhaltene N-Propyl-6-methyl-4-oxo-4H-pyrido[1,2-a]pyrimidin-3-carbonsäureamid-methansulfonsäure schmilzt bei 183-185 °C.

| Analyse für C₁₄H₁₈N₃O₅S | | | |
|---|---|---|---|
| berechnet, %: | C 49,40 | H 5,33 | N 12,34 |
| gefunden, %: | C 49,31 | H 5,25 | N 12,38. |

### Beispiel 29

10 g (0,041 Mol) N-Propyl-6-methyl-4-oxo-4H-pyrido[1,2-a]pyrimidin-3-carbonsäureamid werden unter Erwärmen in 150 ml Äthanol gelöst. Zu der klaren gelben Lösung werden 10 ml 20 m/v-%ige alkoholische Salzsäure gegeben (pH 1). Bei Abkühlen der Lösung scheiden sich Kristalle aus. Man erhält 9,3 g (81,0 %) N-Propyl-6-methyl-4-oxo-4H-pyrido[1,2-a]pyrimidin-3-carbonsäureamid-hydrochlorid, das bei 206-208 °C schmilzt.

| Analyse für C₁₃H₁₆ClN₃O₂ | | | |
|---|---|---|---|
| berechnet, %: | C 55,42 | H 5,72 | N 14,91 |
| gefunden, %: | C 55,47 | H 5,81 | N 14,87. |

### Beispiel 30

3 g (0,011 Mol) N-Cyclopentyl-6-methyl-4-oxo-4H-pyrido[1,2-a]pyrimidin-3-carbonsäureamid werden unter Erwärmen in 45 ml Äthanol gelöst. Zu der klaren gelben Lösung werden tropfenweise 10 ml 20 m/v-%ige alkoholische Salzsäure gegeben (pH = 1). Die sich bei Kühlen des Gemisches abscheidenden Kristalle werden abfiltriert. Man erhält 2,5 g (75,53 %) N-Cyclopentyl-6-methyl-4-oxo-4H-pyrido[1,2-a]pyrimidin-3-carbonsäureamid-hydrochlorid, das bei 205-209 °C schmilzt.

| Analyse für C₁₅H₁₈ClN₃O₂ | | | |
|---|---|---|---|
| berechnet, %: | C 58,54 | H 5,89 | N 13,65 |
| gefunden, %: | C 58,27 | H 6,11 | N 13,69. |

### Beispiel 31

Unter Rühren wird bei Raumtemperatur ein Gemisch aus 1,22 g (0,006 Mol) 7-Methyl-4-oxo-4H-pyrido[1,2-a]pyrimidin-3-carbonsäure, 40 ml Chloroform und 0,84 ml (0,006 Mol) Triäthylamin bereitet und mit einer Salz-Eis-Kältemischung auf unter -10 °C gekühlt. Bei -18 °C wird innerhalb von 10 Minuten tropfenweise die Lösung von 0,46 ml (0,006 Mol) Chlorameisensäuremethylester in 3 ml Chloroform zugegeben. Das Gemisch wird 5 Minuten lang gerührt und dann tropfenweise mit der Lösung von 0,56 g (0,0066 Mol) Cyclopentylamin in 6 ml Chloroform versetzt. Das Gemisch wird bei -10 °C noch eine Stunde lang gerührt und dann über Nacht auf Eis gestellt. Zur Aufarbeitung wird das Gemisch zuerst mit 3x15 ml 5 m/v-%iger Natriumhydrogencarbonatlösung, dann mit 3x15 ml Wasser ausgeschüttelt. Die organische Phase wird über wasserfreiem Natriumsulfat getrocknet und nach dem Filtrieren eingedampft. Man erhält 0,8 g (49,2 %) N-Cyclopentyl-7-methyl-4-oxo-4H-pyrido[1,2-a]pyrimidin-3-carbonsäureamid, das nach Umkristallisieren aus Äthanol bie 162-164 °C schmilzt und als weiße, kristalline Substanz vorliegt.

| Analyse für C₁₅H₁₇N₃O₂ | | | |
|---|---|---|---|
| berechnet, %: | C 66,04 | H 6,31 | N 15,49 |
| gefunden, %: | C 66,19 | H 6,57 | N 15,42 |

### Beispiel 32

Unter Rühren wird bei Raumtemperatur ein Gemisch aus 1,22 g (0,006 Mol) 8-Methyl-4-oxo-4H-pyrido[1,2-a]pyrimidin-3-carbonsäure, 40 ml Chloroform und 0,84 ml (0,006 Mol) Triäthylamin bereitet und mit einer Salz-Eis-Kältemischung auf unter -10 °C gekühlt. Bei -15 °C wird innerhalb von 10 Minuten tropfenweise die Lösung von 0,46 ml (0,006 Mol) Chlorameisensäuremethylester in 3 ml Chloroform zugegeben. Das Gemisch wird 5 Minuten lang gerührt und dann tropfenweise mit der Lösung von 0,56 g (0,0066 Mol) Cyclopentylamin in 6 ml Chloroform versetzt. Das Gemisch wird bei -10 °C noch eine weitere Stunde lang gerührt und dann über Nacht auf Eis gestellt. Zur Aufarbeitung wird das Gemisch zuerst mit 3x15 ml 5m/v-%iger Natriumhydrogencarbonatlösung, dann mit 3x15 ml Wasser ausgeschüttelt. Die organische Phase wird über wasserfreiem Natriumsulfat getrocknet und nach dem Filtrieren eingedampft. Man erhält 0,9 g (55 %) N-Cyclopentyl-8-methyl-4-oxo-4H-pyrido[1,2-a]pyrimidin-3-carbonsäureamid, das nach Umkristallisieren aus Äthanol eine bei 220-222 °C schmelzende kristalline Substanz bildet.

| Analyse für C₁₅H₁₇N₃O₂ | | | |
|---|---|---|---|
| berechnet, %: | C 66,04 | H 6,31 | N 15,49 |
| gefunden, %: | C 66,11 | H 6,38 | N 15,41. |

### Beispiel 33

Unter Rühren wird bei Raumtemperatur ein Gemisch aus 1,22 g (0,006 Mol) 9-Methyl-4-oxo-4H-pyrido[1,2-a]pyrimidin-3-carbonsäure, 40 ml Chloroform und 0,84 ml (0,006 Mol) Triäthylamin bereitet und mit einer Salz-Eis-Kältemischung auf unter -10 °C gekühlt. Bei -15 °C wird innerhalb von 10 Minuten tropfenweise die Lösung von 0,46 ml (0,006 Mol) Chlorameisensäuremethylester in 3 ml Chloroform zugegeben. Das Gemisch wird 5 Minuten lang gerührt und dann innerhalb von 5 Minuten tropfenweise mit der Lösung von 0,56 g (0,0066 Mol) Cyclopentylamin in 6 ml Chloroform versetzt. Das Gemisch wird bei -10 °C noch eine Stunde lang gerührt und dann über Nacht auf Eis gestellt. Zur Aufarbeitung wird das Gemisch mit 3x15 ml 5 m/v-%iger Natriumhydrogencarbonat, dann mit 3x15 ml Wasser gewaschen. Die organische Phase wird über wasserfreiem Natriumsulfat getrocknet und nach dem Filtrieren eingedampft. Man erhält 0,6 g (36,9 %) N-Cyclopentyl-9-methyl-4-oxo-4H-pyrido[1,2-a]pyrimidin-3-carbonsäureamid, das nach Umkristallisieren aus Äthanol eine weiße kristalline Substanz bildet, die bei 170-171 °C schmilzt.

| Analyse für C₁₅H₁₇N₃O₂ | | | |
|---|---|---|---|
| berechnet, %: | C 66,04 | H 6,31 | N 15,49 |
| gefunden, %: | C 65,94 | H 6,27 | N 15,53. |

## Patentansprüche (Patentansprüche für folgende(n) Vertragsstaat(en): AT, BE, CH, DE, FR, GB, IT, LI, LU, NL, SE)

1. Neue 4-Oxo-4H-pyrido[1,2-a]pyrimidin-3-carbonsäureamid-Derivate der allgemeinen Formel (I) 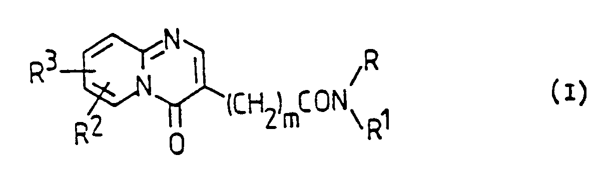 worin
R eine Alkylgruppe mit 1-12 Kohlenstoffatomen, die gegebenfalls durch eine Alkoxycarbonylgruppe mit 1-4 Kohlenstoffatomen substituiert sein kann, ferner eine Cycloalkylgruppe mit 3-9 Kohlenstoffatomen, eine Adamantylgruppe oder eine gegebenenfalls substituierte Phenylgruppe, und
R¹ Wasserstoff oder eine Alkylgruppe mit 1-4 Kohlenstoffatomen bedeuten oder
R und R¹ unter Bildung der Gruppierung der Formel -(CH₂)ₙ-zusammentreten, worin n 4, 5 oder 6 ist,
R² Wasserstoff -oder eine Alkylgruppe mit 1-4 Kohlenstoffatomen,
R³ Wasserstoff oder eine Alkylgruppe mit 1-4 Kohlenstoffatomen, und
m 0 oder 1 bedeuten,
sowie die Salze dieser Verbindungen.

2. Verfahren zur Herstellung neuer 4-Oxo-4H-pyrido[1,2-a]pyrimidin-3-carbonsäureamid-Derivate der allgemeinen Formel (I) 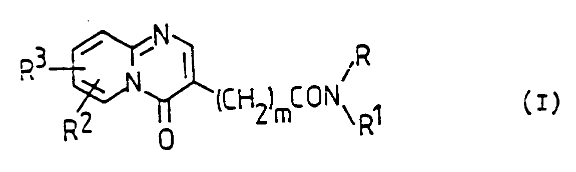 worin
R eine Alkylgruppe mit 1-12 Kohlenstoffatomen, die gegebenfalls durch eine Alkoxycarbonylgruppe mit 1-4 Kohlenstoffatomen substituiert sein kann, ferner eine Cycloalkylgruppe mit 3-9 Kohlenstoffatomen, eine Adamantylgruppe oder eine gegebenenfalls substituierte Phenylgruppe, und
R¹ Wasserstoff oder eine Alkylgruppe mit 1-4 Kohlenstoffatomen bedeuten oder
R und R¹ unter Bildung der Gruppierung der Formel -(CH₂)ₙ-zusammentreten, worin n 4, 5 oder 6 ist,
R² Wasserstoff oder eine Alkylgruppe mit 1-4 Kohlenstoffatomen,
R³ Wasserstoff oder eine Alkylgruppe mit 1-4 Kohlenstoffatomen, und
m 0 oder 1 bedeuten,
sowie der Salze dieser Verbindungen, dadurch gekennzeichnet, daß man
a) 4-Oxo-4H-pyrido[1,2-a]pyrimidin-3-carbonsäureester der allgemeinen Formel (II) 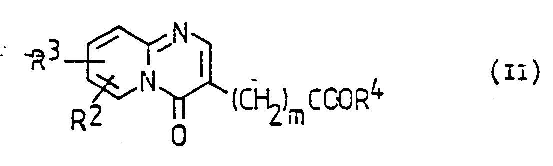 (worin die Bedeutung von R², R³ und m die gleiche wie oben ist und R⁴ für Alkylgruppe mit 1-4 Kohlenstoffatomen steht) mit Aminen der allgemeinen Formel (III) 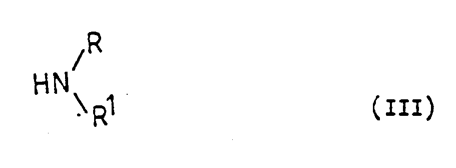 (worin die Bedeutung von R und R¹ die gleiche wie oben ist) oder
b) gemischte 4-Oxo-4H-pyrido[1,2-a]pyrimidin-3-carbonsäure-anhydride der allgemeinen Formel (IV) 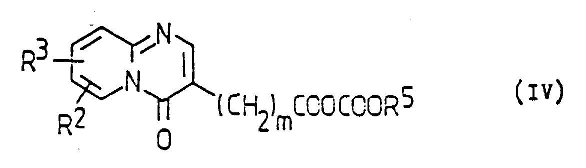 (worin die Bedeutung von R², R³ und m die gleiche wie oben ist, und R⁵ eine Alkylgruppe mit 1-4 Kohlenstoffatomen oder eine Benzylgruppe ist) mit Aminen der allgemeinen Formel (III) umsetzt,
und gewünschtenfalls die erhaltene Verbindung der allgemeinen Formel (I) in an sich bekannter Weise mit einer physiologisch verträglichen Säure zu dem entsprechenden Säureadditionssalz umsetzt oder aus ihren Salz freisetzt und gewünschtenfalls zu einem anderen Salz umsetzt.

3. Arzneimittelpräparate, gekennzeichnet durch einen Gehalt an Verbindungen der allgemeinen Formel (I) gemäß Anspruch 1 oder ihren Salzen.

4. Verwendung der Verbindungen der allgemeinen Formel (I) gemäß Anspruch 1 und ihrer Salze zur Herstellung von insbesondere gastroprotektiv wirkenden Arzneimittelpräparaten.

## Patentansprüche (Patentansprüche für folgende(n) Vertragsstaat(en): ES, GR)

1. Verfahren zur Herstellung neuer 4-Oxo-4H-pyrido[1,2-a]pyrimidin-3-carbonsäureamid-Derivate der allgemeinen Formel (I) 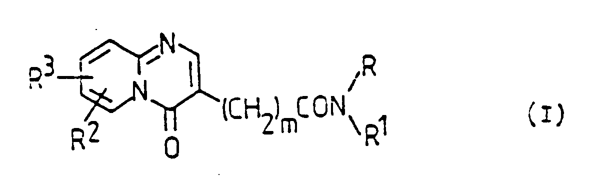 worin
R eine Alkylgruppe mit 1-12 Kohlenstoffatomen, die gegebenfalls durch eine Alkoxycarbonylgruppe mit 1-4 Kohlenstoffatomen substituiert sein kann, ferner eine Cycloalkylgruppe mit 3-9 Kohlenstoffatomen, eine Adamantylgruppe oder eine gegebenenfalls substituierte Phenylgruppe, und
R¹ Wasserstoff oder eine Alkylgruppe mit 1-4 Kohlenstoffatomen bedeuten oder
R und R¹ unter Bildung der Gruppierung der Formel -(CH₂)ₙ-zusammentreten, worin n 4, 5 oder 6 ist,
R² Wasserstoff -oder eine Alkylgruppe mit 1-4 Kohlenstoffatomen,
R³ Wasserstoff oder eine Alkylgruppe mit 1-4 Kohlenstoffatomen, und
m 0 oder 1 bedeuten,
sowie der Salze dieser Verbindungen, dadurch gekennzeichnet, daß man
a) 4-Oxo-4H-pyrido[1,2-a]pyrimidin-3-carbonsäureester der allgemeinen Formel (II) 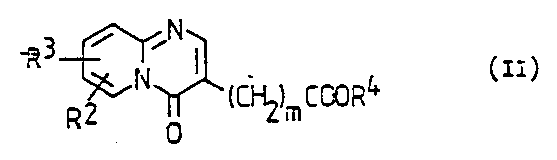 (worin die Bedeutung von R², R³ und m die gleiche wie oben ist und R⁴ für Alkylgruppe mit 1-4 Kohlenstoffatomen steht) mit Aminen der allgemeinen Formel (III) 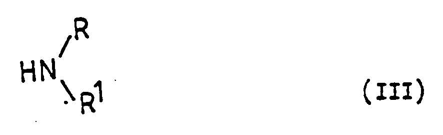 (worin die Bedeutung von R und R¹ die gleiche wie oben ist) oder
b) gemischte 4-Oxo-4H-pyrido[1,2-a]pyimidin-3-carbonsäure-anhydride der allgemeinen Formel (IV) 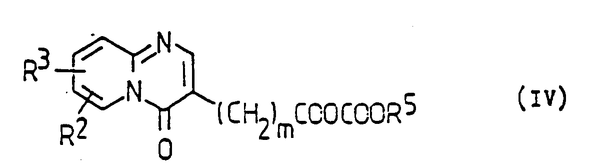 (worin die Bedeutung von R², R³ und m die gleiche, wie oben ist, und R⁵ eine Alkylgruppe mit 1-4 Kohlenstoffatomen oder eine Benzylgruppe ist) mit Aminen der allgemeinen Formel (III) umsetzt,
und gewünschtenfalls die erhaltene Verbindung der allgemeinen Formel (I) in an sich bekannter Weise mit einer physiologisch verträglichen Säure zu dem entsprechenden Säureadditionssalz umsetzt oder aus ihrem Salz freisetzt und gewünschtenfalls zu einem anderen Salz umsetzt.

2. Verwendung der Verbindungen der allgemeinen Formel (I) gemaß Anspruch 1 oder ihren Salzen zur Herstellung von Arzneimittelpräparaten.

3. Verwendung der Verbindungen der allgemeinen Formel (I) gemäß Anspruch 1 und ihrer Salze zur Herstellung von insbesondere gastroprotektiv wirkenden Arzneimittelpräparaten.

## Claims (Claims for the following Contracting State(s): AT, BE, CH, DE, FR, GB, IT, LI, LU, NL, SE)

1. New 4-oxo-4H-pyrido[1,2-a]pyrimidine-3-carboxamide derivatives of general Formula (I) 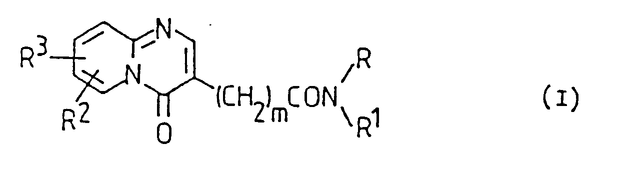 wherein
R represents an alkyl group having 1 to 12 carbon atoms which may optionally be substituted by an alkoxycarbonyl group with 1 to 4 carbon atoms, or is a cycloalkyl group having 3 to 9 carbon atoms, an adamantyl group or an optionally substituted phenyl group, and
R¹ represents hydrogen or an alkyl group having 1 to 4 carbon atoms or
R and R¹ together forms the grouping of formula -(CH₂)ₙ- wherein n is 4, 5 or 6,
R² represents hydrogen or an alkyl group having 1 to 4 carbon atoms,
R³ represents hydrogen or an alkyl group having 1 to 4 carbon atoms, and
m represents 0 or 1,
as well as the salts of these compounds.

2. A process for preparing new 4-oxo-4H-pyrido[1,2-a]pyrimidine-3-carboxamide derivatives of general formula (I) 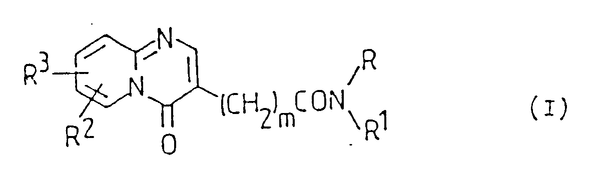 wherein
R represents an alkyl group having 1 to 12 carbon atoms, which may optionally be substituted by an alkoxycarbonyl group having 1 to 4 carbon atoms, or is a cycloalkyl group having 3 to 9 carbon atoms, an adamantyl group or an optionally substituted phenyl group, and
R¹ represents hydrogen or an alkyl group with 1 to 4 carbon atoms or
R and R¹ together form the grouping of formula -(CH₂)ₙ- wherein n represents 4, 5 or 6,
R² represents hydrogen or an alkyl group with 1 to 4 carbon atoms,
R³ represents hydrogen or an alkyl group having 1 to 4 carbon atoms, and
m represents 0 or 1,
as well as the salts of the compounds, characterised in that
a) 4-oxo-4H-pyrido[1,2-a]pyrimidine-3-carboxylate of general Formula (II) 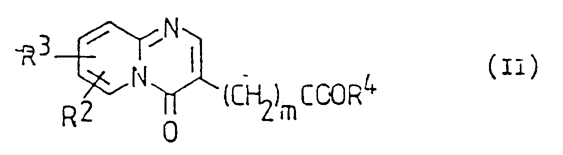 (wherein the meaning of R², R³ and m is the same as given above, and R⁴ represents an alkyl group having 1 to 4 carbon atoms) is reacted with amines of general formula (III) 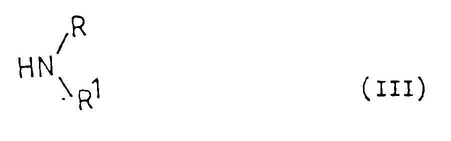 (wherein the meaning of R and R¹ is the same as given above) or
b) mixed 4-oxo-4H-pyrido[1,2-a]pyrimidine-3-carboxylic acid anhydrides of general formula (IV) 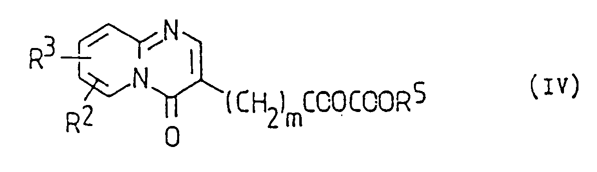 (wherein the meaning of R², R³ and m is the same as given above, and R⁵ is an alkyl group with 1 to 4 carbon atoms or a benzyl group) are reacted with amines of general formula (III), and,
if desired, the resultant compound of general formula (I) is reacted, in a manner known per se, with a physiologically acceptable acid to form the corresponding acid addition salt or is liberated from its salt, and, if desired, is reacted to form another salt.

3. Pharmaceutical preparations, characterised by a content of compounds of general formula (I) according to Claim 1 or the salts thereof.

4. Use of the compounds of general formula (I) according to Claim 1 and the salts thereof for preparing pharmaceutical preparations which have, in particular, a gastro-protective action.

## Claims (Claims for the following Contracting State(s): ES, GR)

1. A process for preparing new 4-oxo-4H-pyrido[1,2-a]pyrimidine-3-carboxamide derivatives of general formula (I) 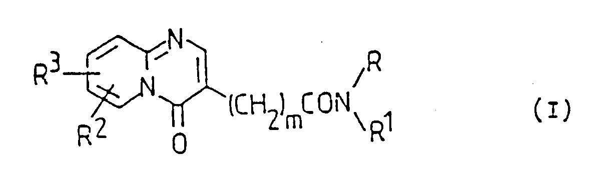 wherein
R represents an alkyl group having 1 to 12 carbon atoms which may optionally be substituted by an alkoxycarbonyl group having 1 to 4 carbon atoms, or is a cycloalkyl group having 3 to 9 carbon atoms, an adamantyl group or an optionally substituted phenyl group, and
R¹ represents hydrogen or an alkyl group having 1 to 4 carbon atoms or
R and R¹ together form the grouping of formula -(CH₂)ₙ- wherein n represents 4, 5 or 6,
R² represents hydrogen or an alkyl group having 1 to 4 carbon atoms,
R³ represents hydrogen or an alkyl group having 1 to 4 carbon atoms, and
m represents 0 or 1,
as well as the salts of these compounds, characterised in that
a) 4-oxo-4H-pyrido[1,2-a]pyrimidine-3-carboxylate of general formula (II) 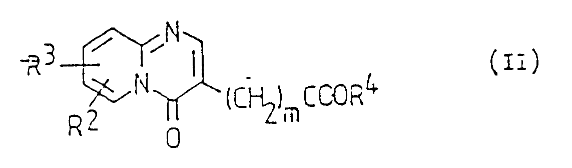 (wherein the meaning of R², R³ and m is the same as given above, and R⁴ represents an alkyl group with 1 to 4 carbon atoms) is reacted with amines of general formula (III) 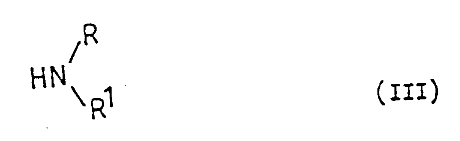 (wherein the meaning of R and R¹ is the same as above), or
b) mixed 4-oxo-4H-pyrido[1,2-a]pyrimidine-3-carboxylic acid anhydrides of general formula (IV) 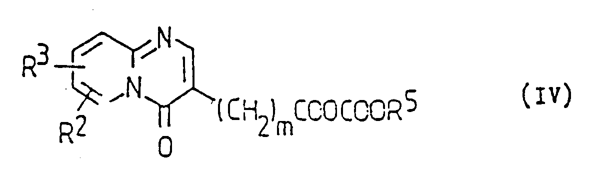 (wherein the meaning of R², R³ and m is the same as given above, and R⁵ is an alkyl group with 1 to 4 carbon atoms or a benzyl group) are reacted with amines of general formula (III),
and, if desired, the resultant compound of general formula (I) is reacted, in a manner known per se, with a physiologically acceptable acid to form the corresponding acid addition salt or is liberated from its salt and, if desired, is reacted to form another salt.

2. Use of the compounds of general formula (I) according to Claim 1 or the salts thereof for preparing pharmaceutical preparations.

3. Use of the compounds of general formula (I) according to Claim 1 and the salts thereof for preparing pharmaceutical preparations which have, in particular, a gastro-protective action.

## Revendications (Revendications pour l'(les) Etat(s) contractant(s) suivant(s): AT, BE, CH, DE, FR, GB, IT, LI, LU, NL, SE)

1. Nouveaux dérivés de 4-oxo-4H-pyrido[1,2-a]pyrimidine-3-carboxamide de formule générale (I) 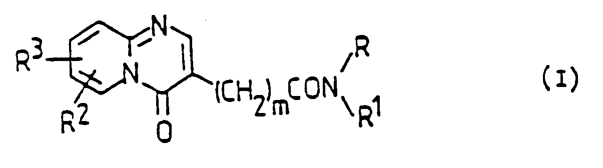 dans laquelle
R représente un groupe alkyle ayant de 1 à 12 atomes de carbone, qui peut éventuellement être substitué par un radical alcoxycarbonyle ayant de 1 à 4 atomes de carbone, ou un groupe cycloalkyle ayant de 3 à 9 atomes de carbone, un groupe adamantyle ou un groupe phényle éventuellement substitué, et
R¹ représente un atome d'hydrogène ou un groupe alkyle ayant de 1 à 4 atomes de carbone, ou
R et R¹ sont réunis pour former un groupement de formule -(CH₂)ₙ-, dans lequel n est 4, 5 ou 6,
R² représente un atome d'hydrogène ou un groupe alkyle ayant de 1 à 4 atomes de carbone,
R³ représente un atome d'hydrogène ou un groupe alkyle ayant de 1 à 4 atomes de carbone, et
m est 0 ou 1,
et sels de ces composés.

2. Procédé pour la préparation de nouveaux dérivés de 4-oxo-4H-pyrido[1,2-a]pyrimidine-3-carboxamide de formule générale (I) 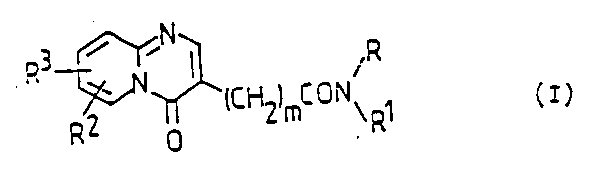 dans laquelle
R représente un groupe alkyle ayant de 1 à 12 atomes de carbone, qui peut éventuellement être substitué par un radical alcoxycarbonyle ayant de 1 à 4 atomes de carbone, ou un groupe cycloalkyle ayant de 3 à 9 atomes de carbone, un groupe adamantyle ou un groupe phényle éventuellement substitué, et
R¹ représente un atome d'hydrogène ou un groupe alkyle ayant de 1 à 4 atomes de carbone, ou
R et R¹ sont réunis pour former un groupement de formule -(CH₂)ₙ-, dans lequel n est 4, 5 ou 6,
R² représente un atome d'hydrogène ou un groupe alkyle ayant de 1 à 4 atomes de carbone,
R³ représente un atome d'hydrogène ou un groupe alkyle ayant de 1 à 4 atomes de carbone, et
m est 0 ou 1,
et des sels de ces composés, caractérisé en ce que
a) on fait réagir des esters d'acides 4-oxo-4H-pyrido[1,2-a]pyrimidine-3-carboxyliques de formule générale (II) 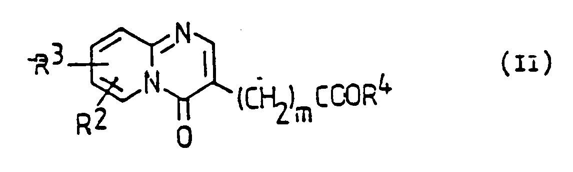 (dans laquelle les significations de R², R³ et m sont les mêmes que ci-dessus et R⁴ représente un groupe alkyle ayant de 1 à 4 atomes de carbone) avec des amines de formule générale (III) 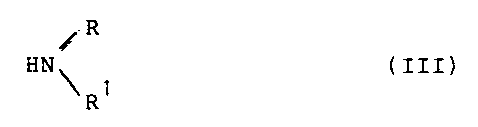 (dans laquelle R et R¹ ont les mêmes significations que ci-dessus), ou
b) on fait réagir des anhydrides 4-oxo-4H-pyrido[1,2-a]pyrimidine-3-carboxyliques mixtes de formule générale (IV) 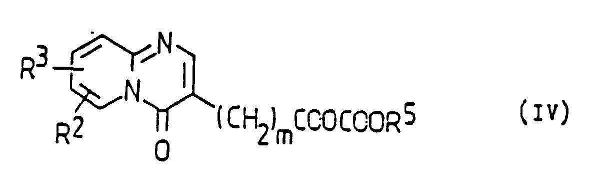 (dans laquelle les significations de R², R³ et m sont les mêmes que ci-dessus, et R⁵ est un groupe alkyle ayant de 1 à 4 atomes de carbone ou le groupe benzyle) avec des amines de formule générale (III),
et, si on le désire, on fait réagir d'une façon connue en soi le composé obtenu, de formule générale (I), avec un acide physiologiquement acceptable, pour aboutir au sel d'addition avec un acide correspondant, ou on le libère à partir d'un de ses sels et, si on le désire, on le convertit en un autre sel.

3. Compositions pharmaceutiques, caractérisées par une teneur en composés de formule générale (I) selon la revendication 1 ou leurs sels.

4. Utilisation des composés de formule générale (I) selon la revendication 1 et de leurs sels, pour la fabrication de médicaments en particulier à action gastroprotectrice.

## Revendications (Revendications pour l'(les) Etat(s) contractant(s) suivant(s): ES, GR)

1. Procédé pour la préparation de nouveaux dérivés de 4-oxo-4H-pyrido[1,2-a]pyrimidine-3-carboxamide de formule générale (I) 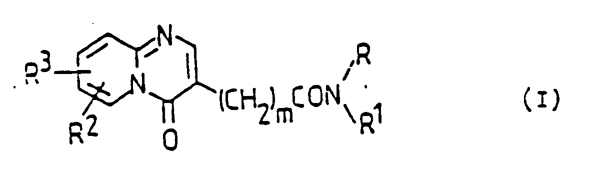 dans laquelle
R représente un groupe alkyle ayant de 1 à 12 atomes de carbone, qui peut éventuellement être substitué par un radical alcoxycarbonyle ayant de 1 à 4 atomes de carbone, ou un groupe cycloalkyle ayant de 3 à 9 atomes de carbone, un groupe adamantyle ou un groupe phényle éventuellement substitué, et
R¹ représente un atome d'hydrogène ou un groupe alkyle ayant de 1 à 4 atomes de carbone, ou
R et R¹ sont réunis pour former un groupement de formule -(CH₂)ₙ-, dans lequel n est 4, 5 ou 6,
R² représente un atome d'hydrogène ou un groupe alkyle ayant de 1 à 4 atomes de carbone,
R³ représente un atome d'hydrogène ou un groupe alkyle ayant de 1 à 4 atomes de carbone, et
m est 0 ou 1,
et des sels de ces composés, caractérisé en ce que
a) on fait réagir des esters d'acides 4-oxo-4H-pyrido[1,2-a]pyrimidine-3-carboxyliques de formule générale (II) 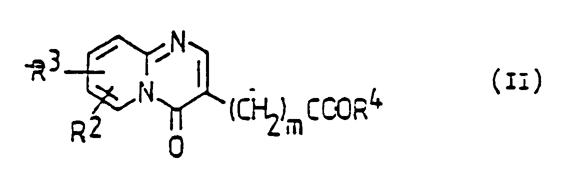 (dans laquelle les significations de R², R³ et m sont les mêmes que ci-dessus et R⁴ représente un groupe alkyle ayant de 1 à 4 atomes de carbone) avec des amines de formule générale (III) 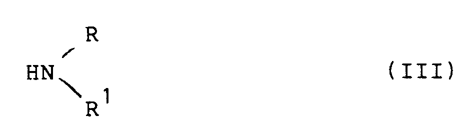 (dans laquelle R et R¹ ont les mêmes significations que ci-dessus), ou
b) on fait réagir des anhydrides 4-oxo-4H-pyrido[1,2-a]pyrimidine-3-carboxyliques mixtes de formule générale (IV) 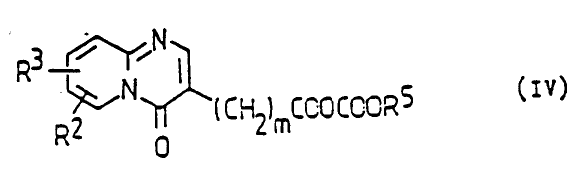 (dans laquelle les significations de R², R³ et m sont les mêmes que ci-dessus, et R⁵ est un groupe alkyle ayant de 1 à 4 atomes de carbone ou le groupe benzyle) avec des amines de formule générale (III),
et, si on le désire, on fait réagir d'une façon connue en soi le composé obtenu, de formule générale (I), avec un acide physiologiquement acceptable, pour aboutir au sel d'addition avec un acide correspondant, ou on le libère à partir d'un de ses sels et, si on le désire, on le convertit en un autre sel.

2. Utilisation des composés de formule générale (I) selon la revendication 1 ou de leurs sels, pour la fabrication de médicaments.

3. Utilisation des composés de formule générale (I) selon la revendication 1 et de leurs sels, pour la fabrication de médicaments en particulier à action gastroprotectrice.
